(19) Europäisches Patentamt
European Patent Office
Office européen des brevets

(11) **EP 1 372 481 B1**

(12) **EUROPEAN PATENT SPECIFICATION**

(45) Date of publication and mention
of the grant of the patent:
**26.10.2005 Bulletin 2005/43**

(51) Int Cl.⁷: **A61B 5/103**

(21) Application number: **02722687.7**

(86) International application number:
**PCT/IT2002/000199**

(22) Date of filing: **27.03.2002**

(87) International publication number:
**WO 2002/078543 (10.10.2002 Gazette 2002/41)**

(54) **SCIENTIFIC PRECISION APPARATUS FOR IDENTIFICATION OF THE SKIN PHOTOTYPE**

GERÄT ZUR IDENTIFIZIERUNG DES HAUTPHOTOTYPS

APPAREIL SCIENTIFIQUE DE PRECISION PERMETTANT D'IDENTIFIER LE PHOTOTYPE DE LA PEAU

(84) Designated Contracting States:
**AT BE CH CY DE DK ES FI FR GB GR IE IT LI LU MC NL PT SE TR**

(30) Priority: **30.03.2001 IT FI20010055**

(43) Date of publication of application:
**02.01.2004 Bulletin 2004/01**

(73) Proprietor: **Villa Borghini S.R.L.**
**52040 Civitella in Val di Chiana (Arezzo) (IT)**

(72) Inventor: **ANGILELLA, Bruno**
**52040 Civitella in Val di Chiana, Arezzo (IT)**

(74) Representative: **Mannucci, Michele et al**
**Ufficio Tecnico Ing.A. Mannucci,**
**Via della Scala 4**
**50123 Firenze (IT)**

(56) References cited:
**WO-A-87/01948**      **WO-A-97/42903**
**DE-C- 19 836 464**      **US-A- 5 995 862**

## Description

Both in the case of exposure to sunlight and in the case of exposure to ultraviolet radiation generated by tanning lamps, one of the most important factors for obtaining the desired aesthetic tanned effect and for avoiding damage to the epidermis is the correct choice of the appropriate protective factor. This depends substantially on the so-called phototype associated with the person who is exposed to radiation. The associated phototype may be precisely determined by means of medical anamnesis on the basis of the color of the eyes, hair, etc. as well as on the basis of examination of the skin. It is obvious that a person who wishes to be exposed to both artifical and natural ultraviolet radiation cannot always undergo medical anamnesis in order to determine the phototype for the purpose of then choosing the appropriate solar protection.

The apparatus which are currently on the market and used also at a university level for scientific research, based on a colorimetric analysis of the epidermis, are unable to obtain satisfactory and reliable results for determination of the phototype since the overall reliability of phototype recognition, in the case of the most precise apparatus currently available, is limited to 71%.

Moreover, the known apparatuses have particular difficulty in distinguishing between the phototypes 2 and 3, the differentiation of which is particularly important for white-skinned people since the association with one or other of the two phototypes results in very different reactions of the epidermis to ultraviolet radiation exposure. Document US 5,995,862 discloses an apparatus comprising all the features of the preamble of claim 1.

It is therefore the object of the present invention to provide an apparatus which allows reliable determination of the associated phototype of a user or person undergoing identification.

These and further objects and advantages, which will be clear to persons skilled in the art from reading of the text which follows, are essentially obtained with a precision apparatus comprising a probe to be applied to the epidermis of the user, with an emitter for emitting electromagnetic radiation towards the epidermis and a receiver for receiving electromagnetic radiation reflected by the epidermis, and a microprocessor processing unit which receives the signal generated by the receiver and, by means of a processing program stored in the unit itself, determines the associated phototype of the user on the basis of the intensity of the signal generated by said receiver, wherein the processing unit takes first and second series of samples of the signal generated by said receiver, calculates a first and a second mean of said first and second series of samples respectively, and determines the associated phototype on the basis of the mean value of said first and second mean. A degree of reliability during measurement equal to 89% is obtained with an apparatus of this type.

Advantageously, the apparatus may comprise means for displaying the phototype determined by said processing unit, for example a display. A printer in addition to or as an alternative to the display may also be envisaged.

The emitter may be an LED which emits electromagnetic radiation for example in the violet and/or ultraviolet range, typically between 400 and 430 nm. The emission is preferably modulated so as to allow electronic filtering of the signal received by the receiver.

Advantageously it is possible to envisage that the phototype (FTO) is determined on the basis of the mean value (I) of the signal from said receiver on the basis of the relation:

$$FTP = I - K_1 - I^2 K_2$$

where $K_1$ and $K_2$ are experimental coefficients.

Further advantageous features and embodiments of the apparatus according to the invention are indicated in the accompanying claims.

The invention will be better understood with reference to the description and the attached drawing which shows a practical non-limiting example of the invention itself. In the drawing:

Fig. 1 shows a block diagram of the apparatus according to the invention;
Figs. 2A and 2B show a flow diagram of the calculation method performed by the microprocessor inside the apparatus according to Fig. 1; and
Figs. 3 and 4 show a schematic perspective view and a schematic longitudinal crosssection of an embodiment of the probe.

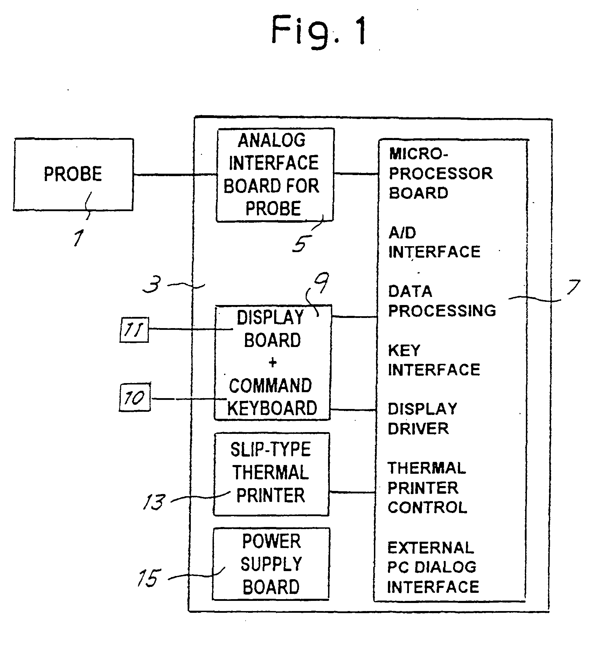

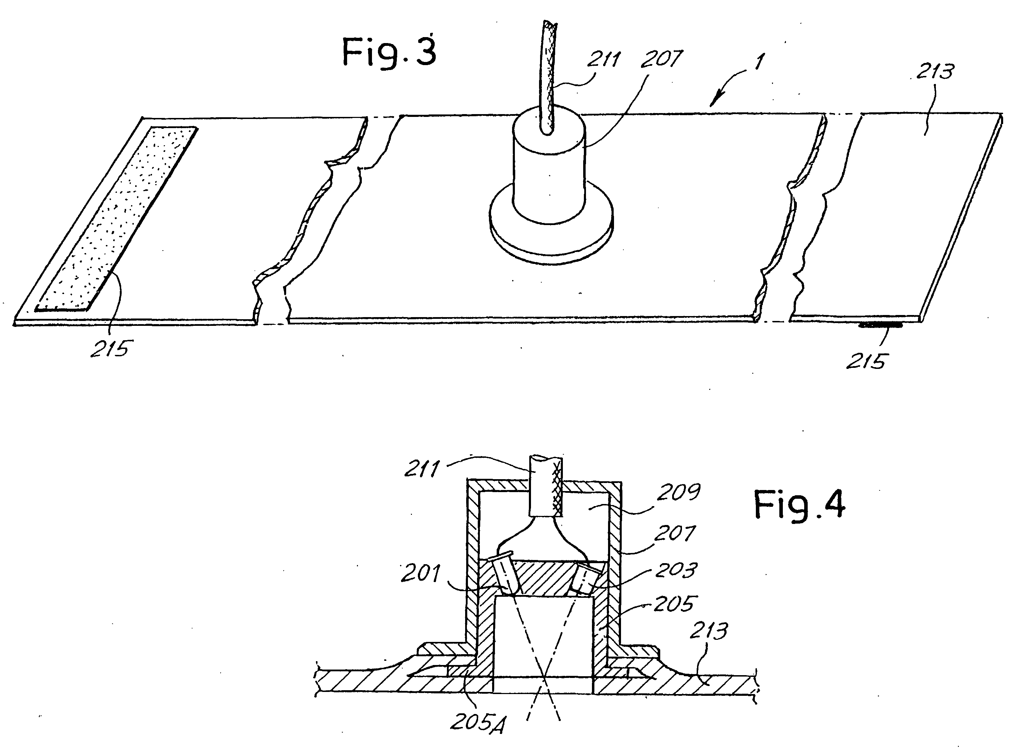

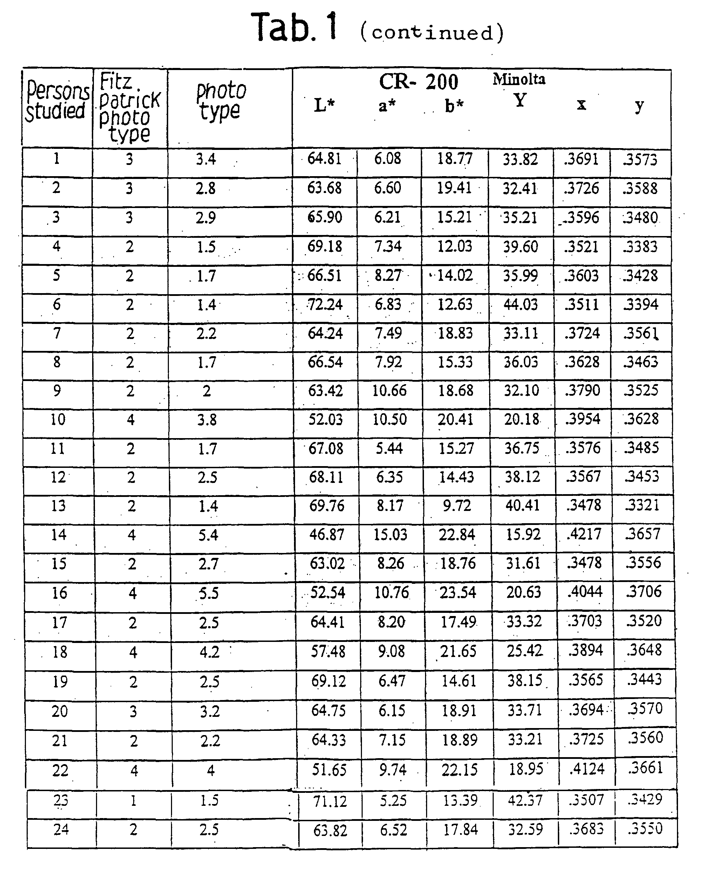

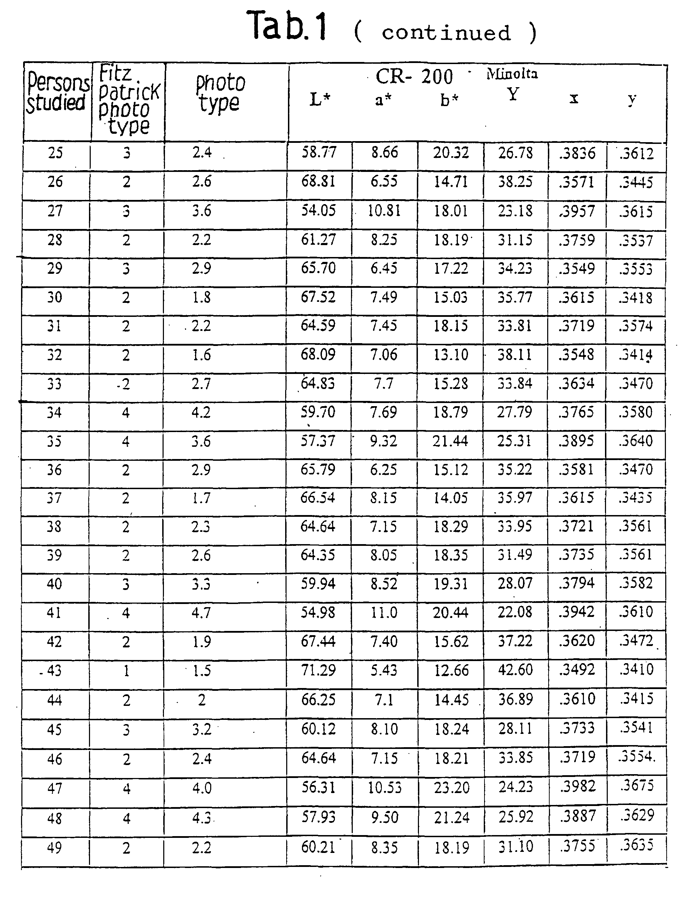

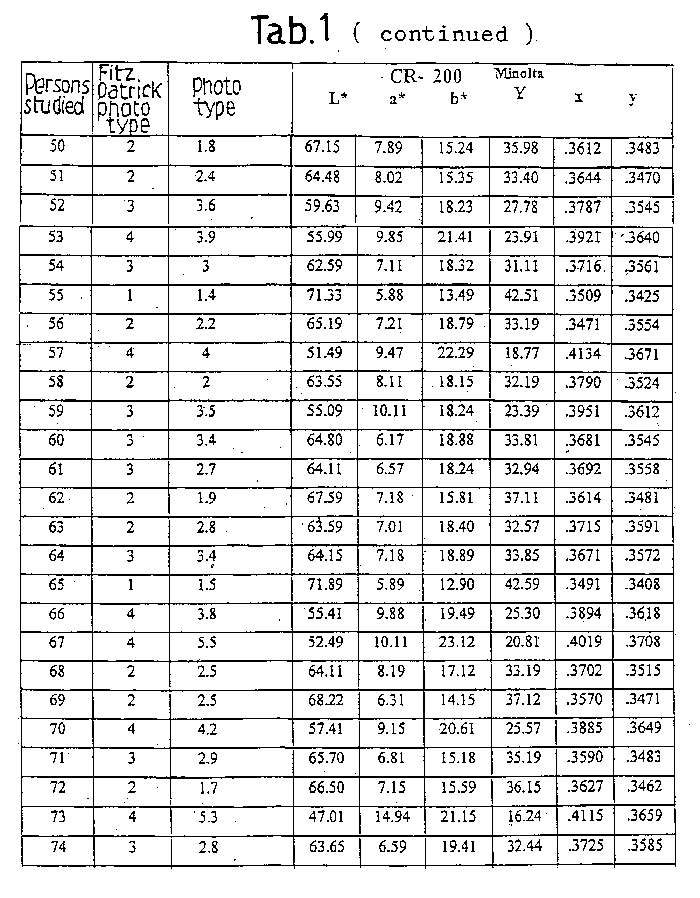

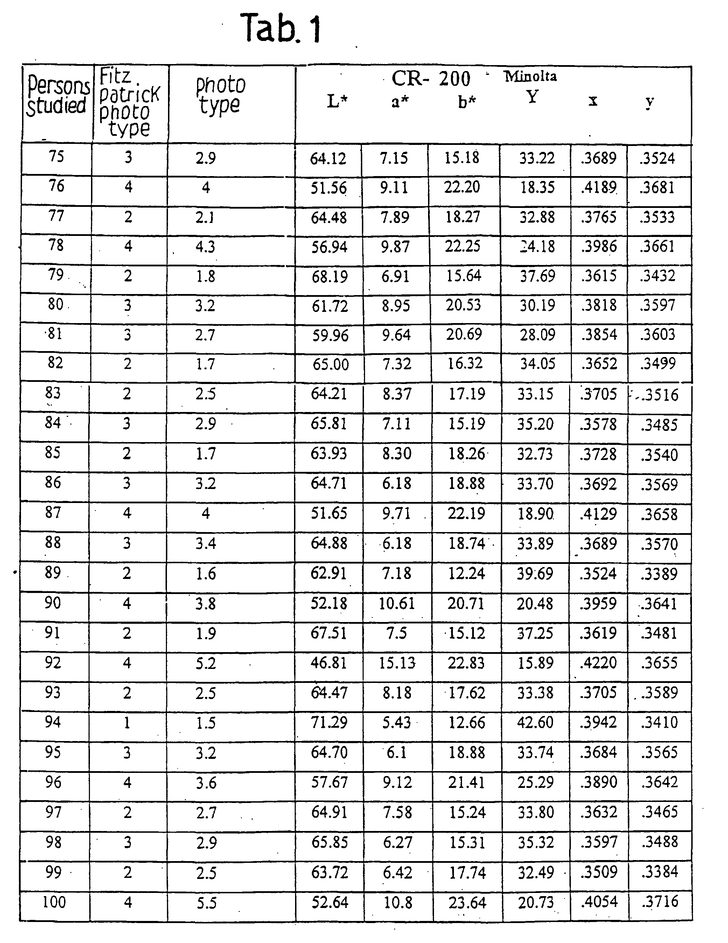

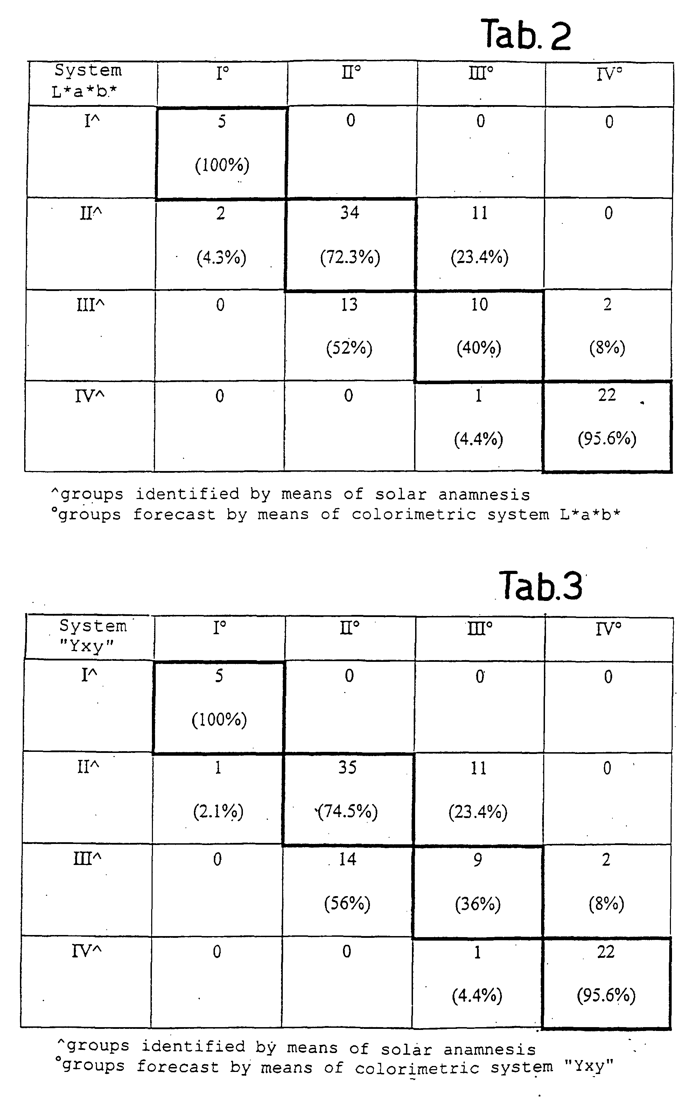

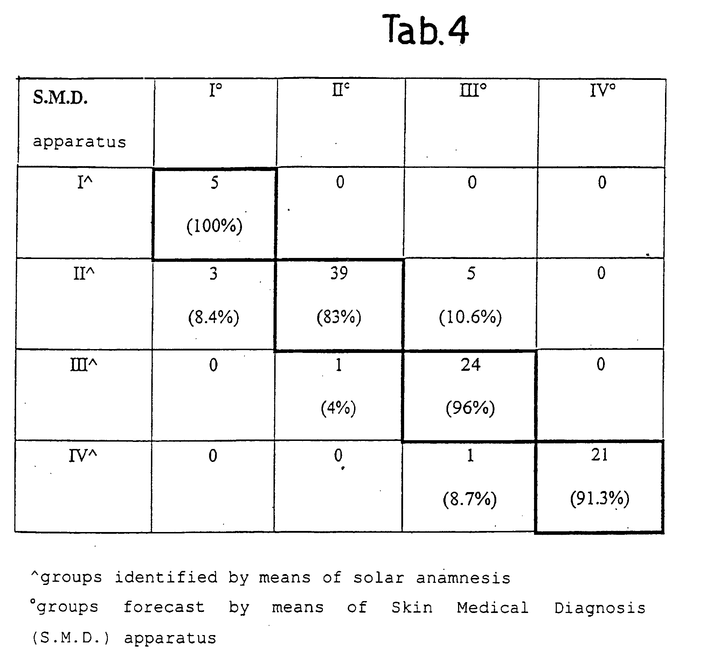

Fig. 1 shows schematically in the form of a block diagram the structure of the apparatus according to the invention. The latter comprises a probe generically denoted by 1 and an apparatus - schematically denoted by 3 - which groups together the electronic boards and the other apparatus managed by the microprocessor of the apparatus. The probe 1, the mechanical structure of which will be described in greater detail with reference to Figs. 3 and 4, essentially consists of a sheet of elastic material inside which a support housing an electronic emitter which emits in the near ultraviolet or in the violet range, and preferably in the range between 400 and 430 nm, is inserted. The beam emitted by the emitter is oriented towards the skin or epidermis of the user who uses the probe when the latter is applied onto the user him/herself. The zone of application of the probe is typically the middle zone of the arm so that the emitter faces a zone of the skin which is not normally subject to tanning.

An electronic receiver which receives the radiation reflected by the skin and emits an electric signal at a voltage proportional to the intensity of the radiation detected is also mounted in the probe support. The sheet-like structure of the probe prevents reading by means of the receiver being altered by the incidence of any ambient light which may pass through the epidermis, in particular in the case of very fair skin.

The probe 1 is interfaced by means of an interface board 5 with a microprocessor board generically denoted by 7. The interface board 5 performs powering of the emitter of the probe 1 and amplification of the return signal generated by the receiver and also dialoguing with the microprocessor board 7. The latter performs processing of the signals from the board 5 and dialogues with the control board of a display and a command keyboard. The said board is denoted by 9, while 10 and 11 schematically denote the keyboard and the display, respectively. The keyboard 10 forms the interface between the apparatus and the user for the introduction of commands, while the display 11 represents the means for showing the results of reading performed by the probe 1 on the user's skin.

The microprocessor board 7 is further interfaced with a printer 13 for the emission of a slip containing the information which the apparatus is programmed to provide.

Finally, 15 denotes a board for powering the apparatus.

The emitter of the probe 1 emits a radiation in the range of 400-430 nm modulated with a square-wave signal at a suitable frequency, typically 5 kHz or at a different value. This allows filtration of the signal received from the receiver electronically, eliminating the effects arising from the incidence, on the receiver, of any electromagnetic radiation with the same length which is not emitted by the emitter. For example, with electronic filtering of the signal captured by the receiver, it is possible to eliminate the influence of the sunlight and also the artificial light which is modulated at the mains frequency.

In order to achieve reliable determination of the phototype, it was found that it is appropriate to perform reading a certain number of times and then perform calculation of the mean value of the readings performed, this also while keeping the probe in a fixed position with respect to the epidermis. Repetition of the reading with sampling of the signal detected and subsequent calculation of the mean value make it possible to avoid inaccuracy in the reading resulting from a multiplicity of spurious factors.

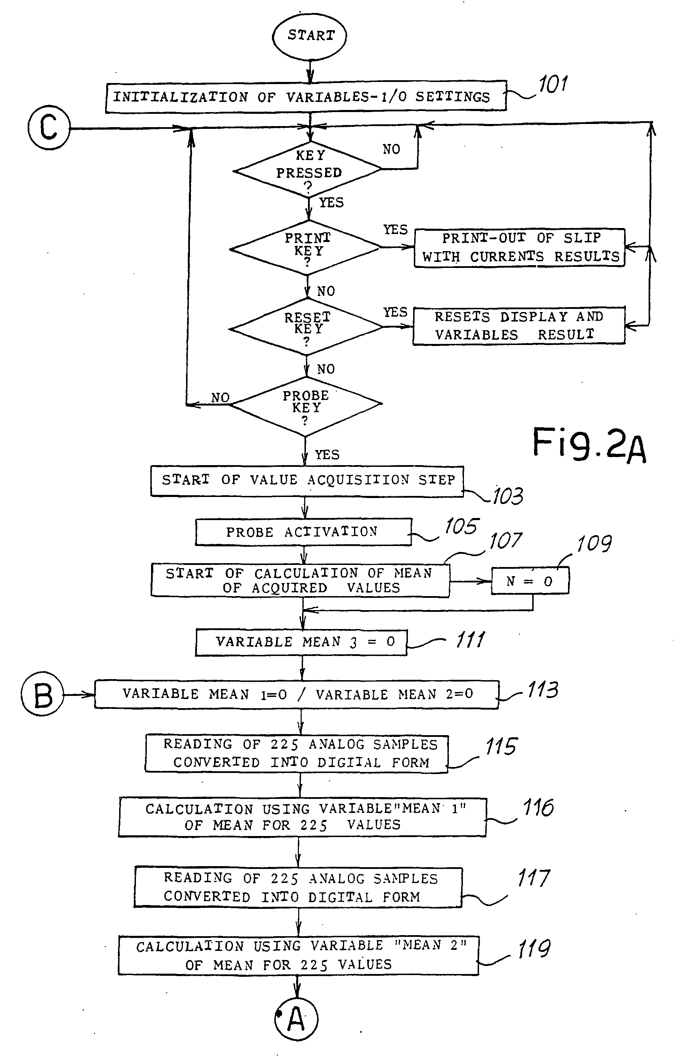

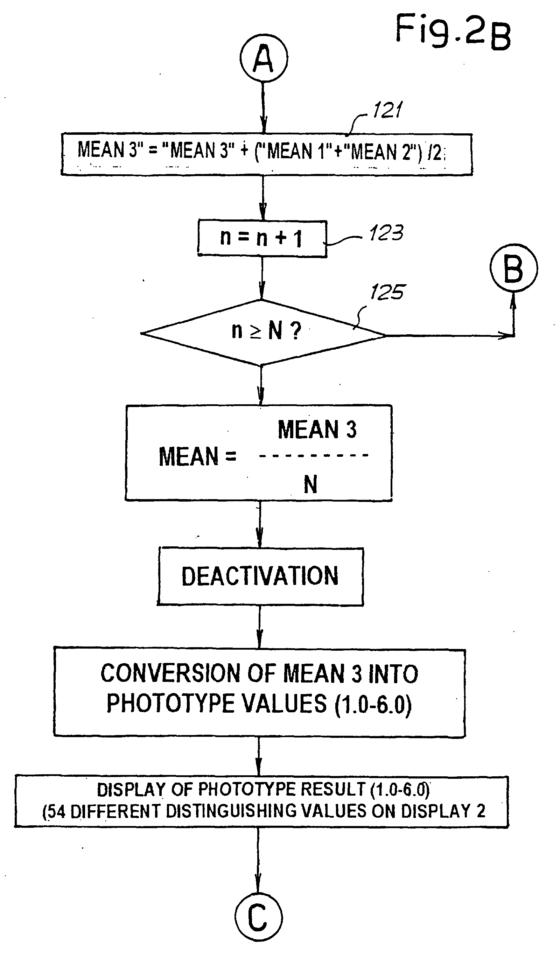

In the example of embodiment described, in order to obtain a high degree of accuracy during determination of the phototype, the microprocessor of the board 7 performs a calculation process which is indicated in the flow diagram of Figs. 2A and 2B, where the initial steps relating to starting of the reading process and the steps of displaying of the results of reading by means of the display 11 and the printer 15 are also shown.

Downstream of the step 101 for initialization of the variables and setting of the inputs and outputs of the boards of the apparatus, four interrogation steps are envisaged for determining whether, on the keyboard 10, the keys for the start of reading, printing of the slip with the results, resetting and reading by means of the probe have been pressed. These keys allow the user to perform a new reading operation, to print out the results of the reading operation performed and reset the apparatus. With the suitable sequence of responses to the four decisional steps, which are clearly shown in the flow diagram, the program reaches the step 103 for the start of the operation involving acquisition of the values read for the phototype. This involves activation of the probe 1 (step 105) and start of the calculation of the mean of the values acquired (step 107) by means of the following operations:

resetting of the value of a counter n (step 109) which counts the number of repetitive cycles of the calculation process;

resetting of the value of the variable "mean3" (step 111);

resetting of the values of the variables "mean1" and "mean2" (step 113);

reading of a first series of samples (in the example 255 samples) of the signal generated by the receiver of the probe 1. The analog signals are converted into digital form (step 115);

calculation of the mean of the 255 values of the first series and storage of the mean as the variable "mean1" (step 116);

reading of a successive second series of 255 analog samples, subsequently converted into digital form (step 117);

calculation of the mean value of the second series of samples and storage as the variable "mean2" (step 119);

calculation of the variable "mean3" using the formula shown in the flow diagram, on the basis of the preceding value of this variable and the mean of the values "mean1" and "mean2" determined in steps 116 and 119 (step 121);

incrementing of the counter n (step 123);

verification of the number of repetitive cycles and conclusion of the repetitive cycle if the counter n is equal to the value N or execution of a new cycle of double readings of 255 samples, calculation of "mean1", "mean2" and "mean3" if the counter n has a value less than N (step 125). The process described hitherto, once the maximum number N of repetitive cycles has been performed, allows storage of a value "mean3" calculated from 510*N sampled values of the signal detected by the receiver of the probe 1. The subsequent operations are performed on the basis of this value. From this value the mean value is calculated by means of division of "mean3" by the value N.

Once reading of the samples and calculation of the mean value have been completed, the probe 1 is switched off and the mean value calculated from the samples read is converted into a phototype value which the apparatus shows on the display in the form of a two-digit number variable from 1.0 to 6.0.

The repetitive reading of a plurality of samples of the signal generated by the receiver of the probe 1 allows a particularly accurate value to be obtained. To avoid reading errors due to the temperature (which may cause drift affecting the receiver) and/or the ambient light, the probe 1 may advantageously assume the configuration shown in Figs. 3 and 4. In these figures, 201 denotes the emitter which is advantageously an LED which emits for example at 430 nm. 203 denotes the receiver, for example a receiving phototransistor. The mutual inclination of the optical axes of the two components is such that they intersect in the vicinity of the zone where the user's epidermis is located.

The emitter 201 and the receiver 203 are mounted on a cup-shaped support 205 inserted inside a cover 207. A cavity for thermal stabilization, which eliminates the reading errors due to variations in the temperature, is defined between the cover 207 and the support 205. The cable 211 for connection of the probe 1 to the apparatus passes through the cover 207.

The cup-shaped support 205 is fixed via its terminal flange 205A to an elastic sheet 213, for example made of silicone material. This sheet is equipped with Velcro strips 215 or other reversible closing means so as to allow fixing to the user's arm. The width of the strip is such as to avoid or reduce to a minimum the ambient ultraviolet or luminous radiation which reaches the optoelectronic components inside the container 205.

The apparatus may be suitable programmed so as to provide, in addition to the result relating to the phetotype determined on the basis of the signal detected by the receiver of the probe 1, also an indication of the protective solar cream most suited to the characteristics of the epidermis (phototype) of the person undergoing measurement.

Results of the university tests

In order to verify the accuracy which the apparatus described above is able to achieve during determination of the phototype, a validation was carried out at the dermatological clinic of the University of Sienna, the corresponding report certifying the tests being held by the proprietors of the present rights. A summary of the validation report is provided below.

A study was carried out on 100 persons belonging to the Caucasian race (60 females and 40 males) with an age ranging between 24 and 38 years (average age of 33 years). The phototype values determined by means of the apparatus described above were compared with the so-called Fitzpatrick medical anamnesis method and with the instrumental results which can be obtained with the CR200 Minolta tristimulus colorimeter®, currently considered to be the most precise apparatus for determining the phototype.

The tristimulus colorimeter is formed by a probe and by a microprocessor. The probe contains a xenon lamp capable of producing a diffused light over the surface to be analyzed. Six silicon photocells, filtered in accordance with that described by the "Commission Internationale d'Eclairage" (CIE), measure the incident and reflected light. The colorimeter expresses results in five different color systems. For the comparison with the experimental data which can be obtained by means of the apparatus according to the present invention, the systems Lab and Yxy available with the Minolta colorimeter® have been considered, since these are the systems most frequently used in the dermatological and cosmetological sector for determination of the phototype.

Before performing the experimental measurements, the solar anamnesis for each person was prepared and, on the basis of this anamnesis, each person was attributed one of the four Caucasian phototypes in accordance with the Fitzpatrick method. After 15 minutes acclimatization in an air-conditioned room at 20°C, the measurements were performed using the apparatus of the present invention and using the Minolta colorimeter® in the region of the middle area of the arm (constitutional color), being careful not to compress the skin causing ischemia and therefore modification of the results of the reading. The measurements were performed using both the apparatus three times and subsequently determination of the mean value was performed.

Since the phototype determined using the Fitzpatrick method is a whole variable of the ordinal type (I, II, III, IV) and vice versa the phototype as supplied by the apparatus of the present invention is a continuous numerical variable, it is not correct to evaluate the correspondence between the two phototypes by means of the Pearson linear correlation coefficient, but it is necessary to determine the discriminating power of the apparatus according to the present invention when distinguishing the various solar phototypes. Firstly, it was verified, by means of the Kolmogorov-Smirnov normality test, that the distributions from which the data relating to each solar phototype were obtained did not differ significantly from the normal distribution (Gaussian distribution). Subsequently, again for each phototype, the experimental values supplied by the apparatus according to the present invention were fitted with the corresponding Gaussian curves. For each of the three pairs of adjacent phototypes, I-II, II-III, III-IV, the corresponding threshold values for intersection between the abovementioned Gaussian curves were determined. It was then determined which of them were correctly classified and which were not correctly classified and the intrinsic percentage errors relating to the superimposed zones of the adjacent Gaussian curves were determined. During evaluation of the correctly classified and incorrectly classified cases, the so-called "leave-one-out" cross-validation technique was used, since this technique allows the effective classification power to be evaluated also in new cases. Once the three thresholds of the apparatus according to the present invention which ensure the best classification result were established, they were interpolated with a straight line so as to adjust to separation values as close as possible to 2, 3 and 4, respectively. In this way reading of the whole part of the new variable converted linearly expresses directly the most probable solar phototype. Finally, restricted zones around the decision zone in which the convenience of defining three new classes of transition solar phototypes I-II, II-III and III-IV could arise were established. The fitted Gaussian distribution method was then repeated also for the color of the skin assessed both using the "Lab" system and the "Yxy" system. Using the Principal Component Analysis technique, each trio of colorimetric values was then represented by the first main component.

Table 1 accompanying the present description shows the determination of the phototype using the Fitzpatrick method and the values supplied both by the apparatus according to the invention and by the tristimulus colorimeter (CR-200 Minolta®) relating to the persons examined. Tables 2, 3 and 4 show the results of the statistical survey. As can be seen, a correspondence of 89% between solar phototype and instrumental phototype determined using the apparatus according to the present invention was recorded. These values are higher than those obtained by means of the tristimulus colorimeter which expressed a correspondence with the Fitzpatrick phototype which was significantly lower (71%).

By way of conclusion, it may be stated that the apparatus according to the invention offers a good degree of reliability in determination of the skin phototype. In fact, its phototype prediction capability is higher (89%) than that which can be obtained by means of the tristimulus colorimeter (71%).

It is understood that the drawing shows only a simplification provided only by way of a practical demonstration of the invention, the forms and arrangements thereof being able to vary without however departing from the scope of the idea underlying the invention itself.

## Claims

1. Precision apparatus for identification of the skin phototype of a user, comprising: a probe (1) to be applied to the epidermis of the user, with an emitter (201) for emitting electromagnetic radiation towards the epidermis and a receiver (203) for receiving electromagnetic radiation reflected by the epidermis; and a microprocessor processing unit (7) which receives the signal generated by the receiver (203) and, by means of a processing program stored in the unit itself, determines the associated phototype of the user on the basis of the intensity of the signal generated by said receiver (203), **char-**

**acterised in that** said processing unit (7) automatically acquires and stores a plurality of samples of the signal generated by said receiver (203) and determines the associated phototype on the basis of a calculation of means of the sampled values, wherein said processing unit (7) performs a first series of readings of the signal of said receiver (203) and calculates a first mean thereof and a second series of readings of the signal and calculates a second mean thereof and **in that** it determines the mean value of said first and said second mean.

2. Apparatus according to one or more of the preceding claims, **characterized in that** the phototype (FTO) is determined on the basis of the mean value (I) of the signal of said receiver on the basis of the relation

$$FTP = I - K_1 - I^2 K_2$$

where $K_1$ and $K_2$ are experimental coefficients.

3. Apparatus according to Claim 1 or 2, **characterized in that** it comprises means (11) for displaying the phototype determined by said processing unit.

4. Apparatus according to Claim 1 or 2 or 3, **characterized in that** said emitter (201) emits an electromagnetic radiation in the violet and/or ultraviolet range.

5. Apparatus according to Claim 4, **characterized in that** said emitter (201) emits an electromagnetic radiation in the range between 400 and 430 nm.

6. Apparatus according to one or more of the preceding claims, **characterized in that** said emitter (201) generates a modulated signal and **in that** the modulated signal reflected by the epidermis of the user is filtered electronically so as to eliminate any continuous components or with modulations at a frequency different from the modulation frequency of the signal emitted by said emitter.

7. Apparatus according to Claim 5, **characterized in that** said signal emitted by the emitter (201) is modulated at a frequency ranging between 1 and 10 kHz.

8. Apparatus according to one or more of the preceding claims, **characterized in that** said processing unit (7) determines the phototype from a mean value of the samples detected by the signal of said receiver, performing the following operations:

   a) resetting of the current value;
   b) execution of a first series of readings of said signal;

c) calculation of the first mean value of said first series of readings;

d) execution of a second series of readings of said signal;

e) calculation of a second mean value of said second series of readings;

f) calculation of the current value as the sum of the preceding current value and the mean of said first mean value and said second mean value;

g) repetition of the steps (b) to (f) for a predetermined number of repetitions;

h) division of the current value by the number of repetitions.

9. Apparatus according to one or more of the preceding claims, **characterized in that** said processing unit (7) determines, on the basis of the phototype associated with the user, a degree of solar protection recommended for a solar cream.

10. Apparatus according to one or more of the preceding claims, **characterized in that** said probe (1) is inserted in a flexible sheet (213) able to be applied to an arm of the user.

11. Apparatus according to one or more of the preceding claims, **characterized in that** said probe (1) has a double-wall structure for preventing the influence of the external temperature on the measurement.

12. Apparatus according to Claim 10 or 11, **characterized in that** said probe (1) comprises a cup-shaped support (205) inside which said emitter (201) and said receiver (203) are inserted, which support (205) is applied to said sheet (213) and is associated with a cover (207) which defines, together with said support (205), a cavity for thermal insulation of the emitter (201) and the receiver (203) with respect to the external environment.

**Patentansprüche**

1. Präzisionsgerät zum Bestimmen des Haut-Lichttyps eines Benutzers, umfassend: eine Sonde (1) zum Anbringen auf der Epidermis des Benutzers, mit einem Sender (201) zum Aussenden elektromagnetischer Strahlung zu der Epidermis und einem Empfänger (203) zum Empfangen von der Epidermis reflektierter elektromagnetischer Strahlung, sowie eine Mikroprozessor-Verarbeitungseinheit (7), die das von dem Empfänger (203) erzeugte Signal empfängt und mittels eines in der Einheit selbst gespeicherten Verarbeitungsprogramms den zugehörigen Lichttyp des Benutzers auf der Basis der Intensität des von dem Empfänger (203) erzeugten Signals bestimmt, **dadurch gekennzeichnet, dass** die Verarbeitungseinheit (7) automatisch eine Anzahl von Abtastwerten des von dem Empfänger (203) erzeugten Signals erfasst und speichert und den zugehörigen Lichttyp anhand einer Berechnung von Mittelwerten der Abtastwerte bestimmt, wobei die Verarbeitungseinheit (7) eine erste Folge von Ablesungen des Signals des Empfängers (203) durchführt und davon einen ersten Mittelwert berechnet und eine zweite Folge von Ablesungen des Signals durchführt und davon einen zweiten Mittelwert berechnet, und dass sie den Mittelwert des ersten und zweiten Mittelwertes bestimmt.

2. Gerät nach einem oder mehreren der vorangehenden Ansprüche, **dadurch gekennzeichnet, dass** der Lichttyp (FTO) auf der Basis des Mittelwertes (I) des Signals von dem Empfänger aufgrund der Beziehung

$$FTP = I - K_1 - I^2 K_2$$

bestimmt, wobei $K_1$ und $K_2$ experimentelle Koeffizienten sind.

3. Gerät nach Anspruch 1 oder 2, **dadurch gekennzeichnet, dass** es Mittel (11) zum Anzeigen des von der Verarbeitungseinheit bestimmten Lichttyps aufweist.

4. Gerät nach Anspruch 1, 2 oder 3, **dadurch gekennzeichnet, dass** der Sender (201) eine elektromagnetische Strahlung im violetten und/oder ultravioletten Bereich aussendet.

5. Gerät nach Anspruch 4, **dadurch gekennzeichnet, dass** der Sender (201) eine elektromagnetische Strahlung im Bereich zwischen 400 und 430 nm aussendet.

6. Gerät nach einem oder mehreren der vorangehenden Ansprüche, **dadurch gekennzeichnet, dass** der Sender (201) ein moduliertes Signal erzeugt und dass das von der Epidermis des Benutzers reflektierte modulierte Signal elektronisch gefiltert wird zum Eliminieren aller kontinuierlichen Komponenten oder von Modulationen mit einer Frequenz, die von der Modulationsfrequenz des von dem Sender ausgesendeten Signals verschieden ist.

7. Gerät nach Anspruch 5, **dadurch gekennzeichnet, dass** das von dem Sender (201) ausgesandte Signal mit einer Frequenz im Bereich zwischen 1 und 10 kHz moduliert ist.

8. Gerät nach einem oder mehreren der vorangehenden Ansprüche, **dadurch gekennzeichnet, dass** die Verarbeitungseinheit (7) den Lichttyp aus einem Mittelwert der von dem Signal des Empfängers detektierten Abtastwerte bestimmt und dabei die folgenden Schritte durchführt:

a) Rücksetzen des aktuellen Wertes;
b) Ausführen einer ersten Folge von Ablesungen des Signals;
c) Berechnen des ersten Mittelwertes der ersten Folge von Ablesungen;
d) Ausführen einer zweiten Folge von Ablesungen des Signals;
e) Berechnen eines zweiten Mittelwertes der zweiten Folge von Ablesungen;
f) Berechnen des aktuellen Wertes als Summe des vorangehenden aktuellen Wertes und des Mittelwertes aus dem ersten Mittelwert und dem zweiten Mittelwert;
g) Wiederholung der Schritte (b) bis (f) für eine vorgegebene Anzahl von Wiederholungen;
h) Teilen des aktuellen Wertes durch die Anzahl der Wiederholungen.

9. Gerät nach einem oder mehreren der vorangehenden Ansprüche, **dadurch gekennzeichnet, dass** die Verarbeitungseinheit (7) auf der Basis des dem Benutzer zugeordneten Lichttyps einen empfohlenen Sonnenschutzfaktor für eine Sonnencreme bestimmt.

10. Gerät nach einem oder mehreren der vorangehenden Ansprüche, **dadurch gekennzeichnet, dass** die Sonde (1) in ein flexibles Band (213) eingesetzt ist, das an dem Arm eines Benutzers anbringbar ist.

11. Gerät nach einem oder mehreren der vorangehenden Ansprüche, **dadurch gekennzeichnet, dass** die Sonde (1) eine doppelwandige Struktur hat, um einen Einfluss der Außentemperatur auf die Messung zu verhindern.

12. Gerät nach Anspruch 10 oder 11, **dadurch gekennzeichnet, dass** die Sonde (1) einen becherförmigen Halter (205) umfasst, in welchem der Sender (201) und der Empfänger (203) eingesetzt sind, wobei der Halter (205) an dem Band (213) angebracht und eine zugeordnete Abdeckung (207) hat, die zusammen mit dem Halter (205) einen Hohlraum für die thermische Isolierung des Senders (201) und des Empfängers (203) von der äußeren Umgebung bildet.

**Revendications**

1. Appareil de précision pour l'identification du phototype de peau d'un utilisateur, comprenant : une sonde (1) devant être appliquée sur l'épiderme de l'utilisateur, avec un émetteur (201) pour émettre un rayonnement électromagnétique en direction de l'épiderme et un récepteur (203) pour recevoir le rayonnement électromagnétique reflété par l'épiderme, et une unité de traitement à microprocesseur (7) qui reçoit le signal généré par le récepteur (203) et qui, au moyen d'un programme de traitement stocké dans l'unité elle-même, détermine le phototype associé de l'utilisateur sur la base de l'intensité du signal généré par ledit récepteur (203), **caractérisé en ce que** ladite unité de traitement (7) acquiert et stocke automatiquement une pluralité d'échantillons de signaux générés par ledit récepteur (203) et détermine le phototype associé sur la base d'un calcul des moyennes des valeurs échantillonnées, dans lequel ladite unité de traitement (7) réalise une première série de lectures du signal dudit récepteur (203) et calcule une première moyenne de celles-ci et une seconde série de lectures du signal et calcule une seconde moyenne de celle-ci et **en ce qu'**elle détermine la valeur moyenne de ladite première et de ladite seconde moyenne.

2. Appareil selon une ou plusieurs des revendications précédentes, **caractérisé en ce que** le phototype (FTO) est déterminé sur la base de la valeur moyenne (I) du signal dudit récepteur sur la base de la relation

$$FTP = I - K_1 - I^2 K_2$$

où $K_1$ et $K_2$ sont des coefficients expérimentaux.

3. Appareil selon la revendication 1 ou 2, **caractérisé en ce qu'**il comprend des moyens (11) pour afficher le phototype déterminé par ladite unité de traitement.

4. Appareil selon la revendication 1 ou 2 ou 3, **caractérisé en ce que** ledit émetteur (201) émet un rayonnement électromagnétique dans le domaine violet et/ou ultraviolet.

5. Appareil selon la revendication 4, **caractérisé en ce que** ledit émetteur (201) émet un rayonnement électromagnétique de 400 à 430 nm.

6. Appareil selon l'une ou plusieurs des revendications précédentes, **caractérisé en ce que** ledit émetteur (201) génère un signal modulé et **en ce que** le signal modulé reflété par l'épiderme de l'utilisateur est filtré électroniquement de manière à éliminer toute composante continue ou avec des modulations à une fréquence différente de la fréquence de modulation du signal émis par ledit émetteur.

7. Appareil selon la revendication 5, **caractérisé en**

**ce que** ledit signal émis par l'émetteur (201) est modulé à une fréquence de 1 à 10 kHz.

8. Appareil selon une ou plusieurs des revendications précédentes, **caractérisé en ce que** ladite unité de traitement (7) détermine le phototype à partir d'une valeur moyenne des échantillons détectés par le signal dudit récepteur, en réalisant les opérations suivantes :

   a) remise à zéro de la valeur en cours ;
   b) exécution d'une première série de lectures dudit signal ;
   c) calcul de la première valeur moyenne de ladite première série de lectures ;
   d) exécution d'une seconde série de lectures dudit signal ;
   e) calcul d'une seconde valeur moyenne de ladite seconde série de lectures ;
   f) calcul de la valeur en cours comme la somme de la précédente valeur en cours et de la moyenne de ladite première valeur moyenne et de ladite seconde valeur moyenne ;
   g) répétition des étapes (b) à (f) pour un nombre prédéterminé de répétitions ;
   h) division de la valeur en cours par le nombre de répétitions.

9. Appareil selon une ou plusieurs des revendications précédentes, **caractérisé en ce que** ladite unité de traitement (7) détermine, sur la base du phototype associé avec l'utilisateur, un niveau de protection solaire recommandé pour une crème solaire.

10. Appareil selon une ou plusieurs des revendications précédentes, **caractérisé en ce que** ladite sonde (1) est insérée dans une feuille flexible (213) pouvant être appliquée sur un bras de l'utilisateur.

11. Appareil selon une ou plusieurs des revendications précédentes, **caractérisé en que** ladite sonde (1) a une structure à double paroi pour empêcher la température extérieure d'influencer les mesures.

12. Appareil selon la revendication 10 ou 11, **caractérisé en ce que** ladite sonde (1) comprend un support creux (205) à l'intérieur duquel ledit émetteur (201) et ledit récepteur (203) sont insérés, lequel support (205) est appliqué sur ladite feuille (213) et est associé avec un couvercle (207) qui définit, conjointement avec ledit support (205), une cavité pour l'isolation thermique de l'émetteur (201) et du récepteur (203) par rapport à l'environnement extérieur.

# Fig. 1

PROBE

*1*

*3*

ANALOG
INTERFACE
BOARD FOR
PROBE

*5*

*9*

[11]

DISPLAY
BOARD
+
COMMAND
KEYBOARD

[10]

SLIP-TYPE
THERMAL
PRINTER

*13*

POWER
SUPPLY
BOARD

*15*

MICRO-
PROCESSOR
BOARD

A/D
INTERFACE

DATA
PROCESSING

KEY
INTERFACE

DISPLAY
DRIVER

THERMAL
PRINTER
CONTROL

EXTERNAL
PC DIALOG
INTERFACE

*7*

START

INITIALIZATION OF VARIABLES-1/0 SETTINGS — *101*

Ⓒ

KEY PRESSED ? — NO

YES

PRINT KEY ? — YES → PRINT-OUT OF SLIP WITH CURRENTS RESULTS

NO

RESET KEY ? — YES → RESETS DISPLAY AND VARIABLES RESULT

NO

NO ← PROBE KEY ?

YES

START OF VALUE ACQUISITION STEP — *103*

PROBE ACTIVATION — *105*

START OF CALCULATION OF MEAN OF ACQUIRED VALUES — *107* → N = 0 — *109*

VARIABLE MEAN 3 = 0 — *111*

Ⓑ → VARIABLE MEAN 1=0 / VARIABLE MEAN 2=0 — *113*

READING OF 225 ANALOG SAMPLES CONVERTED INTO DIGITAL FORM — *115*

CALCULATION USING VARIABLE "MEAN 1" OF MEAN FOR 225 VALUES — *116*

READING OF 225 ANALOG SAMPLES CONVERTED INTO DIGITAL FORM — *117*

CALCULATION USING VARIABLE "MEAN 2" OF MEAN FOR 225 VALUES — *119*

Ⓐ

Fig.2A

10

# Fig.2B

(A)

*121*

MEAN 3" = "MEAN 3" + ("MEAN 1"+"MEAN 2") /2

n = n + 1  *123*

(B)

n ≥ N ?  *125*

$$MEAN = \frac{MEAN\ 3}{N}$$

DEACTIVATION

CONVERSION OF MEAN 3 INTO
PHOTOTYPE VALUES (1.0-6.0)

DISPLAY OF PHOTOTYPE RESULT (1.0-6.0)
(54 DIFFERENT DISTINGUISHING VALUES ON DISPLAY 2

(C)

Fig.3

Fig.4

# Tab. 1 (continued)

| Persons Studied | Fitz patrick photo type | photo type | CR- 200 | | | Minolta | | |
|---|---|---|---|---|---|---|---|---|
| | | | L* | a* | b* | Y | x | y |
| 1 | 3 | 3.4 | 64.81 | 6.08 | 18.77 | 33.82 | .3691 | .3573 |
| 2 | 3 | 2.8 | 63.68 | 6.60 | 19.41 | 32.41 | .3726 | .3588 |
| 3 | 3 | 2.9 | 65.90 | 6.21 | 15.21 | 35.21 | .3596 | .3480 |
| 4 | 2 | 1.5 | 69.18 | 7.34 | 12.03 | 39.60 | .3521 | .3383 |
| 5 | 2 | 1.7 | 66.51 | 8.27 | 14.02 | 35.99 | .3603 | .3428 |
| 6 | 2 | 1.4 | 72.24 | 6.83 | 12.63 | 44.03 | .3511 | .3394 |
| 7 | 2 | 2.2 | 64.24 | 7.49 | 18.83 | 33.11 | .3724 | .3561 |
| 8 | 2 | 1.7 | 66.54 | 7.92 | 15.33 | 36.03 | .3628 | .3463 |
| 9 | 2 | 2 | 63.42 | 10.66 | 18.68 | 32.10 | .3790 | .3525 |
| 10 | 4 | 3.8 | 52.03 | 10.50 | 20.41 | 20.18 | .3954 | .3628 |
| 11 | 2 | 1.7 | 67.08 | 5.44 | 15.27 | 36.75 | .3576 | .3485 |
| 12 | 2 | 2.5 | 68.11 | 6.35 | 14.43 | 38.12 | .3567 | .3453 |
| 13 | 2 | 1.4 | 69.76 | 8.17 | 9.72 | 40.41 | .3478 | .3321 |
| 14 | 4 | 5.4 | 46.87 | 15.03 | 22.84 | 15.92 | .4217 | .3657 |
| 15 | 2 | 2.7 | 63.02 | 8.26 | 18.76 | 31.61 | .3478 | .3556 |
| 16 | 4 | 5.5 | 52.54 | 10.76 | 23.54 | 20.63 | .4044 | .3706 |
| 17 | 2 | 2.5 | 64.41 | 8.20 | 17.49 | 33.32 | .3703 | .3520 |
| 18 | 4 | 4.2 | 57.48 | 9.08 | 21.65 | 25.42 | .3894 | .3648 |
| 19 | 2 | 2.5 | 69.12 | 6.47 | 14.61 | 38.15 | .3565 | .3443 |
| 20 | 3 | 3.2 | 64.75 | 6.15 | 18.91 | 33.71 | .3694 | .3570 |
| 21 | 2 | 2.2 | 64.33 | 7.15 | 18.89 | 33.21 | .3725 | .3560 |
| 22 | 4 | 4 | 51.65 | 9.74 | 22.15 | 18.95 | .4124 | .3661 |
| 23 | 1 | 1.5 | 71.12 | 5.25 | 13.39 | 42.37 | .3507 | .3429 |
| 24 | 2 | 2.5 | 63.82 | 6.52 | 17.84 | 32.59 | .3683 | .3550 |

# Tab.1 ( continued )

| Persons Studied | Fitz patrick photo type | photo type | CR- 200 Minolta | | | | | |
|---|---|---|---|---|---|---|---|---|
| | | | L* | a* | b* | Y | x | y |
| 25 | 3 | 2.4 | 58.77 | 8.66 | 20.32 | 26.78 | .3836 | .3612 |
| 26 | 2 | 2.6 | 68.81 | 6.55 | 14.71 | 38.25 | .3571 | .3445 |
| 27 | 3 | 3.6 | 54.05 | 10.81 | 18.01 | 23.18 | .3957 | .3615 |
| 28 | 2 | 2.2 | 61.27 | 8.25 | 18.19 | 31.15 | .3759 | .3537 |
| 29 | 3 | 2.9 | 65.70 | 6.45 | 17.22 | 34.23 | .3549 | .3553 |
| 30 | 2 | 1.8 | 67.52 | 7.49 | 15.03 | 35.77 | .3615 | .3418 |
| 31 | 2 | 2.2 | 64.59 | 7.45 | 18.15 | 33.81 | .3719 | .3574 |
| 32 | 2 | 1.6 | 68.09 | 7.06 | 13.10 | 38.11 | .3548 | .3414 |
| 33 | -2 | 2.7 | 64.83 | 7.7 | 15.28 | 33.84 | .3634 | .3470 |
| 34 | 4 | 4.2 | 59.70 | 7.69 | 18.79 | 27.79 | .3765 | .3580 |
| 35 | 4 | 3.6 | 57.37 | 9.32 | 21.44 | 25.31 | .3895 | .3640 |
| 36 | 2 | 2.9 | 65.79 | 6.25 | 15.12 | 35.22 | .3581 | .3470 |
| 37 | 2 | 1.7 | 66.54 | 8.15 | 14.05 | 35.97 | .3615 | .3435 |
| 38 | 2 | 2.3 | 64.64 | 7.15 | 18.29 | 33.95 | .3721 | .3561 |
| 39 | 2 | 2.6 | 64.35 | 8.05 | 18.35 | 31.49 | .3735 | .3561 |
| 40 | 3 | 3.3 | 59.94 | 8.52 | 19.31 | 28.07 | .3794 | .3582 |
| 41 | 4 | 4.7 | 54.98 | 11.0 | 20.44 | 22.08 | .3942 | .3610 |
| 42 | 2 | 1.9 | 67.44 | 7.40 | 15.62 | 37.22 | .3620 | .3472 |
| 43 | 1 | 1.5 | 71.29 | 5.43 | 12.66 | 42.60 | .3492 | .3410 |
| 44 | 2 | 2 | 66.25 | 7.1 | 14.45 | 36.89 | .3610 | .3415 |
| 45 | 3 | 3.2 | 60.12 | 8.10 | 18.24 | 28.11 | .3733 | .3541 |
| 46 | 2 | 2.4 | 64.64 | 7.15 | 18.21 | 33.85 | .3719 | .3554. |
| 47 | 4 | 4.0 | 56.31 | 10.53 | 23.20 | 24.23 | .3982 | .3675 |
| 48 | 4 | 4.3 | 57.93 | 9.50 | 21.24 | 25.92 | .3887 | .3629 |
| 49 | 2 | 2.2 | 60.21 | 8.35 | 18.19 | 31.10 | .3755 | .3635 |

# Tab.1 ( continued )

| Persons studied | Fitz. patrick photo type | photo type | CR- 200 | | | Minolta Y | x | y |
|---|---|---|---|---|---|---|---|---|
| | | | L* | a* | b* | | | |
| 50 | 2 | 1.8 | 67.15 | 7.89 | 15.24 | 35.98 | .3612 | .3483 |
| 51 | 2 | 2.4 | 64.48 | 8.02 | 15.35 | 33.40 | .3644 | .3470 |
| 52 | 3 | 3.6 | 59.63 | 9.42 | 18.23 | 27.78 | .3787 | .3545 |
| 53 | 4 | 3.9 | 55.99 | 9.85 | 21.41 | 23.91 | .3921 | .3640 |
| 54 | 3 | 3 | 62.59 | 7.11 | 18.32 | 31.11 | .3716 | .3561 |
| 55 | 1 | 1.4 | 71.33 | 5.88 | 13.49 | 42.51 | .3509 | .3425 |
| 56 | 2 | 2.2 | 65.19 | 7.21 | 18.79 | 33.19 | .3471 | .3554 |
| 57 | 4 | 4 | 51.49 | 9.47 | 22.29 | 18.77 | .4134 | .3671 |
| 58 | 2 | 2 | 63.55 | 8.11 | 18.15 | 32.19 | .3790 | .3524 |
| 59 | 3 | 3.5 | 55.09 | 10.11 | 18.24 | 23.39 | .3951 | .3612 |
| 60 | 3 | 3.4 | 64.80 | 6.17 | 18.88 | 33.81 | .3681 | .3545 |
| 61 | 3 | 2.7 | 64.11 | 6.57 | 18.24 | 32.94 | .3692 | .3558 |
| 62 | 2 | 1.9 | 67.59 | 7.18 | 15.81 | 37.11 | .3614 | .3481 |
| 63 | 2 | 2.8 | 63.59 | 7.01 | 18.40 | 32.57 | .3715 | .3591 |
| 64 | 3 | 3.4 | 64.15 | 7.18 | 18.89 | 33.85 | .3671 | .3572 |
| 65 | 1 | 1.5 | 71.89 | 5.89 | 12.90 | 42.59 | .3491 | .3408 |
| 66 | 4 | 3.8 | 55.41 | 9.88 | 19.49 | 25.30 | .3894 | .3618 |
| 67 | 4 | 5.5 | 52.49 | 10.11 | 23.12 | 20.81 | .4019 | .3708 |
| 68 | 2 | 2.5 | 64.11 | 8.19 | 17.12 | 33.19 | .3702 | .3515 |
| 69 | 2 | 2.5 | 68.22 | 6.31 | 14.15 | 37.12 | .3570 | .3471 |
| 70 | 4 | 4.2 | 57.41 | 9.15 | 20.61 | 25.57 | .3885 | .3649 |
| 71 | 3 | 2.9 | 65.70 | 6.81 | 15.18 | 35.19 | .3590 | .3483 |
| 72 | 2 | 1.7 | 66.50 | 7.15 | 15.59 | 36.15 | .3627 | .3462 |
| 73 | 4 | 5.3 | 47.01 | 14.94 | 21.15 | 16.24 | .4115 | .3659 |
| 74 | 3 | 2.8 | 63.65 | 6.59 | 19.41 | 32.44 | .3725 | .3585 |

# Tab. 1

| Persons Studied | Fitz patrick photo type | photo type | CR- 200 | Minolta | | | | |
| --- | --- | --- | --- | --- | --- | --- | --- | --- |
| | | | L* | a* | b* | Y | x | y |
| 75 | 3 | 2.9 | 64.12 | 7.15 | 15.18 | 33.22 | .3689 | .3524 |
| 76 | 4 | 4 | 51.56 | 9.11 | 22.20 | 18.35 | .4189 | .3681 |
| 77 | 2 | 2.1 | 64.48 | 7.89 | 18.27 | 32.88 | .3765 | .3533 |
| 78 | 4 | 4.3 | 56.94 | 9.87 | 22.25 | 24.18 | .3986 | .3661 |
| 79 | 2 | 1.8 | 68.19 | 6.91 | 15.64 | 37.69 | .3615 | .3432 |
| 80 | 3 | 3.2 | 61.72 | 8.95 | 20.53 | 30.19 | .3818 | .3597 |
| 81 | 3 | 2.7 | 59.96 | 9.64 | 20.69 | 28.09 | .3854 | .3603 |
| 82 | 2 | 1.7 | 65.00 | 7.32 | 16.32 | 34.05 | .3652 | .3499 |
| 83 | 2 | 2.5 | 64.21 | 8.37 | 17.19 | 33.15 | .3705 | .3516 |
| 84 | 3 | 2.9 | 65.81 | 7.11 | 15.19 | 35.20 | .3578 | .3485 |
| 85 | 2 | 1.7 | 63.93 | 8.30 | 18.26 | 32.73 | .3728 | .3540 |
| 86 | 3 | 3.2 | 64.71 | 6.18 | 18.88 | 33.70 | .3692 | .3569 |
| 87 | 4 | 4 | 51.65 | 9.71 | 22.19 | 18.90 | .4129 | .3658 |
| 88 | 3 | 3.4 | 64.88 | 6.18 | 18.74 | 33.89 | .3689 | .3570 |
| 89 | 2 | 1.6 | 62.91 | 7.18 | 12.24 | 39.69 | .3524 | .3389 |
| 90 | 4 | 3.8 | 52.18 | 10.61 | 20.71 | 20.48 | .3959 | .3641 |
| 91 | 2 | 1.9 | 67.51 | 7.5 | 15.12 | 37.25 | .3619 | .3481 |
| 92 | 4 | 5.2 | 46.81 | 15.13 | 22.83 | 15.89 | .4220 | .3655 |
| 93 | 2 | 2.5 | 64.47 | 8.18 | 17.62 | 33.38 | .3705 | .3589 |
| 94 | 1 | 1.5 | 71.29 | 5.43 | 12.66 | 42.60 | .3942 | .3410 |
| 95 | 3 | 3.2 | 64.70 | 6.1 | 18.88 | 33.74 | .3684 | .3565 |
| 96 | 4 | 3.6 | 57.67 | 9.12 | 21.41 | 25.29 | .3890 | .3642 |
| 97 | 2 | 2.7 | 64.91 | 7.58 | 15.24 | 33.80 | .3632 | .3465 |
| 98 | 3 | 2.9 | 65.85 | 6.27 | 15.31 | 35.32 | .3597 | .3488 |
| 99 | 2 | 2.5 | 63.72 | 6.42 | 17.74 | 32.49 | .3509 | .3384 |
| 100 | 4 | 5.5 | 52.64 | 10.8 | 23.64 | 20.73 | .4054 | .3716 |

## Tab. 2

| System L*a*b* | I° | II° | III° | IV° |
|---|---|---|---|---|
| I^ | 5 <br><br>(100%) | 0 | 0 | 0 |
| II^ | 2 <br><br>(4.3%) | 34 <br><br>(72.3%) | 11 <br><br>(23.4%) | 0 |
| III^ | 0 | 13 <br><br>(52%) | 10 <br><br>(40%) | 2 <br><br>(8%) |
| IV^ | 0 | 0 | 1 <br><br>(4.4%) | 22 <br><br>(95.6%) |

^groups identified by means of solar anamnesis
°groups forecast by means of colorimetric system L*a*b*

## Tab.3

| System "Yxy" | I° | II° | III° | IV° |
|---|---|---|---|---|
| I^ | 5 <br><br>(100%) | 0 | 0 | 0 |
| II^ | 1 <br><br>(2.1%) | 35 <br><br>(74.5%) | 11 <br><br>(23.4%) | 0 |
| III^ | 0 | 14 <br><br>(56%) | 9 <br><br>(36%) | 2 <br><br>(8%) |
| IV^ | 0 | 0 | 1 <br><br>(4.4%) | 22 <br><br>(95.6%) |

^groups identified by means of solar anamnesis
°groups forecast by means of colorimetric system "Yxy"

# Tab.4

| S.M.D. apparatus | I° | II° | III° | IV° |
|---|---|---|---|---|
| I^ | 5 (100%) | 0 | 0 | 0 |
| II^ | 3 (8.4%) | 39 (83%) | 5 (10.6%) | 0 |
| III^ | 0 | 1 (4%) | 24 (96%) | 0 |
| IV^ | 0 | 0 | 1 (8.7%) | 21 (91.3%) |

^groups identified by means of solar anamnesis

°groups forecast by means of Skin Medical Diagnosis (S.M.D.) apparatus